# EUROPEAN PATENT SPECIFICATION

(11) **EP 1 631 571 B1**
(45) Date of publication and mention of the grant of the patent: **23.04.2008**
(21) Application number: 04741560.9
(22) Date of filing: 13.05.2004
(51) Int. Cl.: C07D 471/14

(54) **NOVEL INTERMEDIATE FOR THE PREPARATION OF THERAPEUTICALLY ACTIVE IMIDAZOPYRIDINES**
NEUE ZWISCHENPRODUKTE FÜR DIE HERSTELLUNG THERAPEUTISCH WIRKSAMER IMIDAZOPYRIDINE
NOUVEL INTERMEDIAIRE POUR LA PREPARATION D'IMIDAZOPYRIDINES A ACTIVITE THERAPEUTIQUE

(30) Priority: 14.05.2003 EP 03010785
(43) Date of publication of application: 08.03.2006
(73) Proprietor: Nycomed GmbH, 78467 Konstanz (DE)
(72) Inventor: ZIMMERMANN, Peter, 78467 Konstanz (DE); SCHEUFLER, Christian, 78234 Engen-Neuhausen (DE); KOHL, Bernhard, 78465 Konstanz (DE); MUELLER, Bernd, 78467 Konstanz (DE)
(74) Representative: Wild, Robert
(86) International application number: PCT/EP2004/050780
(87) International publication number: WO 2004/101566

(56) References cited:
- WO-A-00/17200
- WO-A-01/72756
- US-A- 3 749 721

## Description

### Technical field

The invention relates to novel compounds which are used in the pharmaceutical industry as valuable intermediates for the preparation of active compounds.

### Prior art

The international patent applications WO 98/42707, WO 00/17200, WO 00/26217, WO 00/63211 WO 01/72756, WO 01/72754, WO 01/72757, WO 02/34749, WO 03/016310, WO 03/014120 and WO 03/014123 disclose tricyclic imidazopyridine derivatives having a very specific substitution pattern, which should be suitable for the treatment of gastric and intestinal disorders, and certain processes for their preparation.

### Description of the Invention

The invention relates to a new process for the production of the tricyclic imidazopyridine derivatives mentioned above, and to valuable intermediates used in said processes.

The invention thus relates in a first aspect to the 1,5-naphthalene disulfonic acid addition salt of the compound of the formula 1

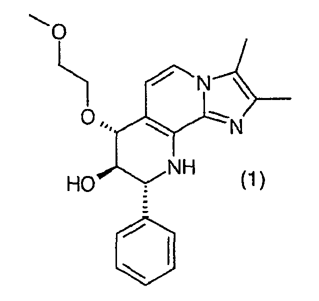

It is known to the person skilled in the art that the compound according to the invention, if it is isolated, for example, in crystalline form, can contain various amounts of solvents. The invention therefore also comprises all solvates and in particular all hydrates of the 1,5-naphthalene disulfonic acid addition salt of the compound of the formula 1.

The 1,5-naphthalene disulfonic acid addition salt of the compound of the formula 1 according to the invention can be prepared from the compounds of the formula 2 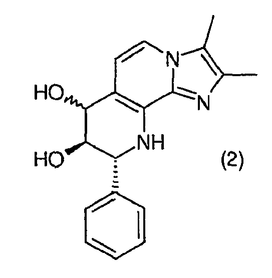 as shown in scheme 1 by reaction with the 1,5-naphthalene disulfonic acid of the formula 3 and with 2-methoxy-ethanol of the formula 4. Reaction of a cis/trans diol mixture or of either the cis or the trans compound of the diol of the formula 2 leads exclusively to the precipitation of the desired 1,5-naphthalene disulfonic acid addition salt of the compound of the formula 1. Under acidic reaction conditions the chiral centres in 7 position of the diol of the formula 2 and of the compound of the formula 1 epimerise and an equilibrium between the cis and the trans compounds is established. The 1,5-naphthalene disulfonic acid addition salt of the compound of the formula 1 is the only compound which precipitates from these reaction mixtures.

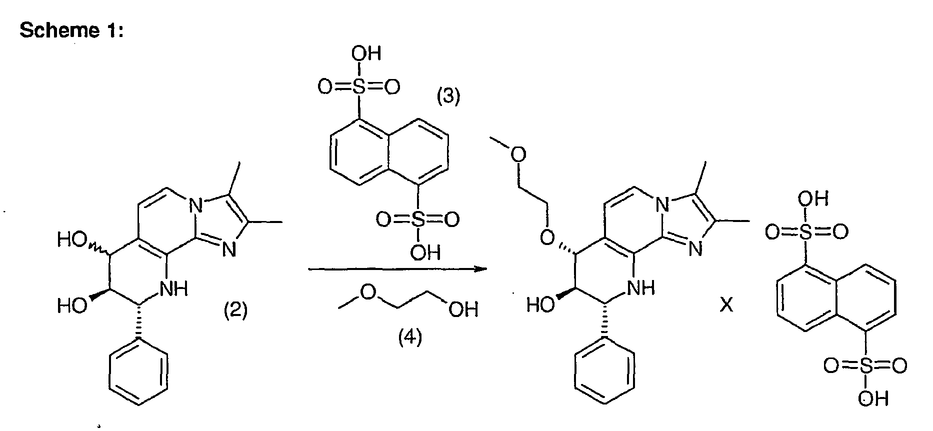

1,5-Naphthalene disulfonic acid and the compound of the formula 1 form acid addition salts according to the invention with different molar ratios of 1,5-naphthalene disulfonic acid and of the compound of the formula 1.

Preferred acid addition salts according to the invention are those, in which the molar ratio of 1,5-naphthalene disulfonic acid and the compound of the formula 1 is between 1 : 1 and 2 : 1.

Particularly preferred acid addition salts according to the invention are those, in which the molar ratio of 1,5-naphthalene disulfonic acid and the compound of the formula 1 is between 1 : 1 and 1.2 : 1.

The reaction shown in scheme 1 is carried out either without solvent or in a suitable solvent or in a suitable mixture of solvents. The reaction is preferably carried out in a suitable solvent.

The reaction is preferably carried out in the absence of water.

Preferred solvents for the reaction shown in scheme 1 are ketones such as for example acetone, methylethylketone or isobutylmethylketone or ethers such as for example diethylether, diisopropylether or methyltertbutylether.

A particularly preferred solvent for the reaction shown in scheme 1 is isobutylmethylketone.

The invention further relates to the use of the 1,5-naphthalene disulfonic acid addition salt of the compound of the formula 1 for the preparation of the compound of the formula 1 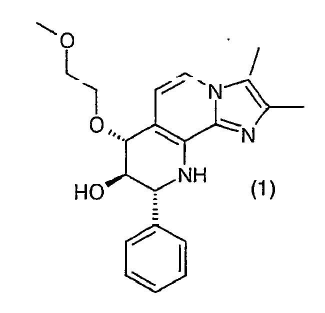 which is known from the international patent application WO 00/17200, and its salts.

Suitable salts of compounds of the formula 1 in this regard are especially all acid addition salts. Particular mention may be made of the pharmacologically tolerable salts of the inorganic and organic acids customarily used in pharmacy. Those suitable are water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 3-hydroxy-2-naphthoic acid, where the acids are employed in salt preparation - depending on whether a mono- or polybasic acid is concerned and depending on which salt is desired - in an equimolar quantitative ratio or one differing therefrom.

These salts of the compound of the formula 1 are mentioned in the patent application WO 00/17200 and they are not suitable for the process according to the present invention as shown in scheme 1.

The process of the use of the 1,5-naphthalene disulfonic acid addition salt of the compound of the formula 1 according to the invention for the preparation of the compound of the formula 1 is carried out, as shown in scheme 2, such that the 1,5-naphthalene disulfonic acid addition salt of the compound of the formula 1 is reacted with a base and then worked up.

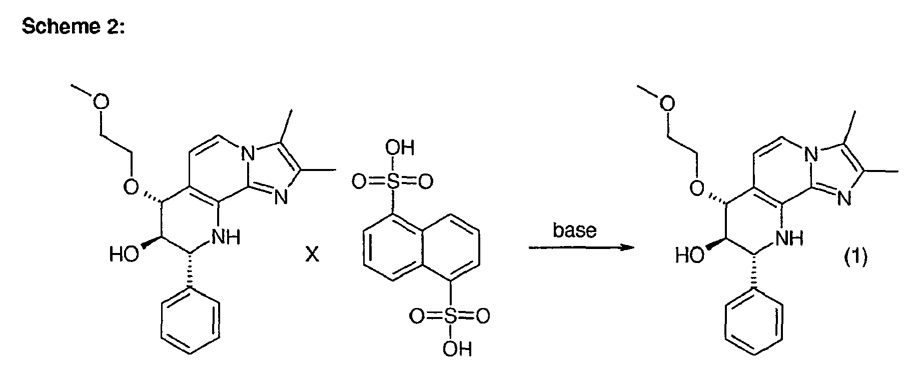

Starting diol compounds of the formula 2 for the preparation of the 1,5-naphthalene disulfonic acid addition salt of the compound of the formula 1 according to the invention are known (see WO 00/17200).

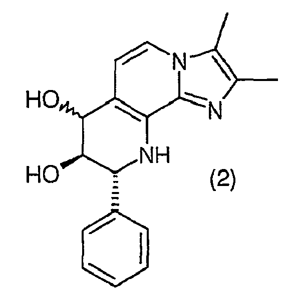

In the patent application WO 00/17200 the trans diol compound of the formula 2a 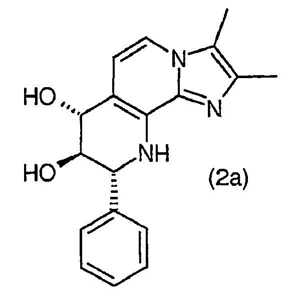 is used for the preparation of the compound of the formula 1 by reaction of the compound of the formula 2a with 2-methoxy-ethanol and sulfuric acid. This reaction described in WO 00/17200 using sulfuric acid leads to the formation of the undesired compound of the formula 1 b in amounts equal or even higher than that of the desired compound of the formula 1.

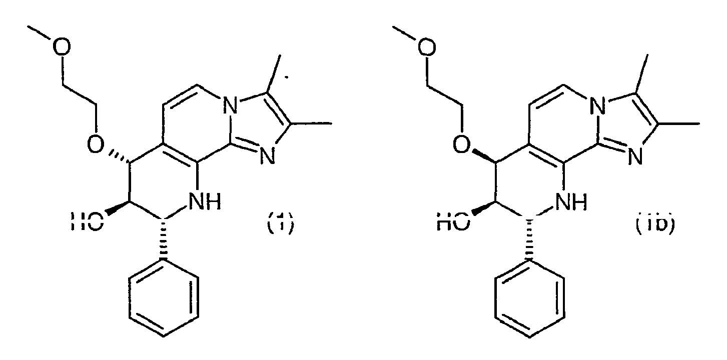

The disadvantage of the formation of the undesired compound of the formula 1 b in the etherification reaction of compounds of the formula 2 described in WO 00/17200 can be overcome with the present invention. The use of 1,5-naphthalene-disulfonic acid instead of sulfuric acid in the reaction of compounds of the formula 2 with 2-methoxy-ethanol leads in a high yield to the exclusive formation of the 1,5-naphthalene disulfonic acid addition salt of the compound of the formula 1, which can be converted in a subsequent step into the compound of the formula 1.

The following examples serve to illustrate the invention further without restricting it. The abbreviation min stands for minute(s), h for hour(s) and m. p. for melting point.

### Examples

### 1,5 naphthalene disulfonic acid salt of [(7R,8R,9R)-2,3-Dimethyl-8-hydroxy-7-(2-methoxyethoxy)-9-phenyl-7,8,9,10-tetrahydro-imidazo-[1,2-h][1,7]-naphthyridine]

18.8 g (52.2 mmol) 1,5-Naphthalene-disulfonic acid tetrahydrate were dissolved in 100 ml of 2-methoxy-ethanol. 70 ml of the 2-methoxy-ethanol were distilled off in vacuo (50 mbar) to remove the crystal water. A solution of 5.00 g (16.2 mmol) of a mixture of (7R,8R,9R)-7,8-dihydroxy-2,3-dimethyl-9-phenyl-7,8,9,10-tetrahydro-imidazo[1,2-h][1,7]-naphthyridine and (7S,8R,9R)-7,8-dihydroxy-2,3-dimethyl-9-phenyl-7,8,9,10-tetrahydro-imidazo[1,2-h][1,7]-naphthyridine in 20 ml of 2-methoxy-ethanol was added. The mixture was diluted with 60 ml of isobutylmethylketone and then stirred at room temperature for 30 h. The resulting suspension was cooled to 0°C, stirred for 2 h at that temperature and then filtered. The residue was washed with 50 ml of isobutylmethylketone and then dried in vacuo to afford 8.9 g (13.6 mmol, yield: 84 %) of the title compound.
m. p.: 147°C (sintering), 180°C (decomposition)
¹H-NMR (200 MHz, DMSO): δ = 2,40 (s, 3H); 2,43 (s, 3H); 3,27 (s, 3H); 3,46 (m, 2H); 3,86 (m, 2H); 3,96 (m, 1H); 4,53 (d, 1H); 4,66 (d, 1H); 7,35-7,49 (m, 9H); 7,00 7,08 (m, 3H); 8,00 (d, 2H)

### Use of the 1 5 naphthalene disulfonic acid salt of the compound of the formula 1 according to the invention for the preparation of the compound of the formula 1

8.9 g (13.6 mmol) of the 1,5 naphthalene disulfonic acid salt of [(7R,8R,9R)-2,3-dimethyl-8-hydroxy-7-(2-methoxyethoxy)-9-phenyl-7,8,9,10-tetrahydro-imidazo-[1,2-h][1,7]-naphthyridine] were added to a diluted, aqueous solution of sodium hydroxide (40.4 mmol). The free base was then extracted with dichloromethane and the organic phase concentrated in vacuo. The residue was triturated with ethyl acetate to afford 3.4 g (9.3 mmol, yield: 68%) of [(7R,8R,9R)-2,3-Dimethyl-8-hydroxy-7-(2-methoxyethoxy)-9-phenyl-7,8,9,10-tetrahydro-imidazo-[1,2-h][1,7]-naphthyridine].

### Commercial utility

The 1,5-naphthalene disulfonic acid addition salt of the compound of the formula 1 is a valuable intermediate for the preparation of the active compound of the formula 1 and its salts, which are disclosed in the international patent application WO 00/17200.

## Claims

1. The 1,5-naphthalene disulfonic acid addition salt of the compound of the formula 1

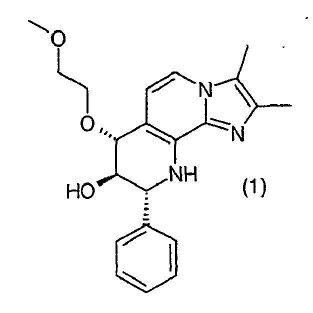

2. The 1,5-naphthalene disulfonic acid addition salt of the compound of the formula 1 according to claim 1, in which the molar ratio of 1,5-naphthalene disulfonic acid and the compound of the formula 1 is between 1 : 1 and 2 : 1.

3. The 1,5-naphthalene disulfonic acid addition salt of the compound of the formula 1 according to claim 1, in which the molar ratio of 1,5-naphthalene disulfonic acid and the compound of the formula 1 is between 1 : 1 and 1.2 : 1.

4. The use of the 1,5-naphthalene disulfonic acid addition salt of the compound of the formula 1 as claimed in claim 1, for the preparation of the compound of the formula 1 and its salts.

5. A process for the preparation of the 1,5-naphthalene disulfonic acid addition salt as claimed in claim 1, which comprises reaction of a compound of the formula 2 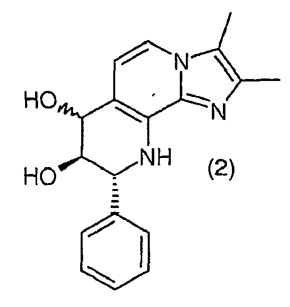 with the 1,5-naphthalene disulfonic acid of the formula 3 and an alcohol of the formula 4 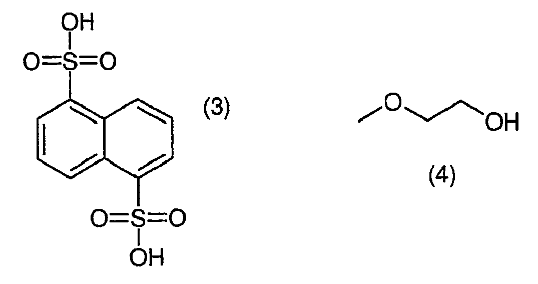 and then working up.

6. A process as claimed in claim 5, which is carried out in a suitable solvent.

7. A process as claimed in claim 5, which is carried out in a ketone selected from the group of acetone, methylethylketone or isobutylmethylketone or in an ether selected from the group of diethylether, diisopropylether or methyltertbutylether as solvent.

8. A process as claimed in claim 5, which is carried out in isobutylmethylketone as solvent.

## Patentansprüche

1. 1,5-Naphthalindisulfonsäure-Additionssalz der Verbindung der Formel 1

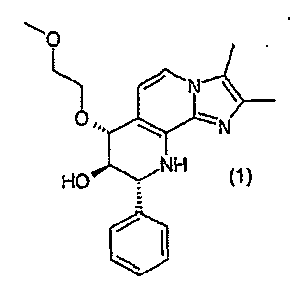

2. 1,5-Naphthalindisulfonsäure-Additionssalz der Verbindung der Formel 1 nach Anspruch 1, wobei das Molverhältnis von 1,5-Naphthalindisulfonsäure zur Verbindung der Formel 1 zwischen 1:1 und 2:1 liegt.

3. 1,5-Naphthalindisulfonsäure-Additionssalz der Verbindung der Formel 1 nach Anspruch 1, wobei das Molverhältnis von 1,5-Naphthalindisulfonsäure zur Verbindung der Formel 1 zwischen 1:1 und 1,2:1 liegt.

4. Verwendung des 1,5-Naphthalindisulfonsäure-Additionssalzes der Verbindung der Formel 1 nach Anspruch 1 zur Herstellung der Verbindung der Formel 1 und ihrer Salze.

5. Verfahren zur Herstellung des 1,5-Naphthalindisulfonsäure-Additionssalzes nach Anspruch 1, bei dem man eine Verbindung der Formel 2 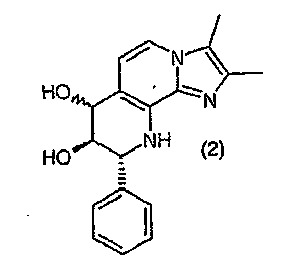 mit der 1,5-Naphthalindisulfonsäure der Formel 3 und einem Alkohol der Formel 4 umsetzt 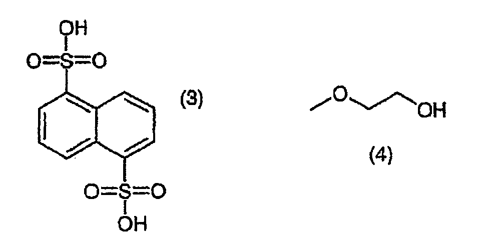 und dann aufarbeitet.

6. Verfahren nach Anspruch 5, durchgeführt in einem geeigneten Lösungsmittel.

7. Verfahren nach Anspruch 5, durchgeführt in einem Keton ausgewählt aus der Gruppe bestehend aus Aceton, Methylethylketon und Isobutylmethylketon oder in einem Ether ausgewählt aus der Gruppe bestehend aus Diethylether, Diisopropylether und Methyl-tert.-butylether als Lösungsmittel.

8. Verfahren nach Anspruch 5, durchgeführt in Isobutylmethylketon als Lösungsmittel.

## Revendications

1. Sel d'addition d'acide 1,5-naphtalènedisulfonique du composé de formule 1

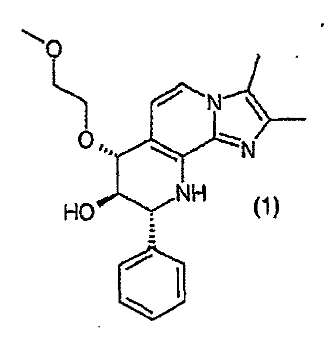

2. Sel d'addition d'acide 1,5-naphtalènedisulfonique du composé de formule 1 selon la revendication 1, **caractérisé en ce que** le rapport molaire entre l'acide 1,5-naphtalènedisulfonique et le composé de formule 1 est compris entre 1:1 et 2:1.

3. Sel d'addition d'acide 1,5-naphtalènedisulfonique du composé de formule 1 selon la revendication 1, **caractérisé en ce que** le rapport molaire entre l'acide 1,5-naphtalènedisulfonique et le composé de formule 1 est compris entre 1:1 et 1,2:1.

4. Utilisation du sel d'addition d'acide 1,5-naphtalènedisulfonique du composé de formule 1 selon la revendication 1, pour la préparation du composé de formule 1 et de ses sels.

5. Procédé de préparation du sel d'addition d'acide 1,5-naphtalènedisulfonique selon la revendication 1, **caractérisé en ce qu'**il comprend la réaction d'un composé de formule 2 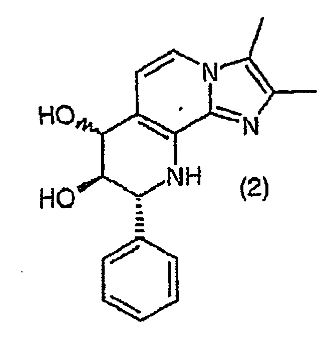 avec l'acide 1,5-naphtalènedisulfonique de formule 3 et un alcool de formule 4 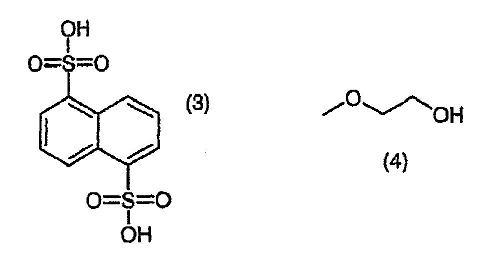 puis les traitements classiques.

6. Procédé selon la revendication 5, **caractérisé en ce qu'**il est effectué dans un solvant convenable.

7. Procédé selon la revendication 5, qui est effectué dans une cétone choisie dans le groupe comprenant l'acétone, la méthyléthylcétone ou l'isobutylméthylcétone ou dans un éther choisi dans le groupe comprenant le diéthyléther, le diisopropyléther ou le méthyltertbutyléther comme solvant.

8. Procédé selon la revendication 5, **caractérisé en ce qu'**il est effectué dans l'isobutylméthylcétone comme solvant.
